# EUROPÄISCHE PATENTSCHRIFT

(11) **EP 0 942 702 B1**
(45) Veröffentlichungstag und Bekanntmachung des Hinweises auf die Patenterteilung: **26.06.2002**
(21) Anmeldenummer: 97912169.6
(22) Anmeldetag: 10.10.1997
(51) Int. Cl.: A61F 15/00, B65D 75/34, B65D 75/42

(54) **VORRATSEINRICHTUNG FÜR MEDIZINISCHE TUPFER**
STORAGE DEVICE FOR MEDICAL SWABS
SYSTEME DE RESERVOIR POUR TAMPONS A USAGE MEDICAL

(30) Priorität: 27.11.1996 DE 29620636 U
(43) Veröffentlichungstag der Anmeldung: 22.09.1999
(73) Patentinhaber: Lohmann GmbH & Co. KG, 56567 Neuwied (DE)
(72) Erfinder: FELLINGER, Andreas, D-56459 Ailertchen (DE)
(74) Vertreter: Flaccus, Rolf-Dieter, Dr.
(86) Internationale Anmeldenummer: EP9705609
(87) Internationale Veröffentlichungsnummer: WO9823238

(56) Entgegenhaltungen:
- WO-A-95/18046
- DE-A- 3 128 547
- FR-A- 2 722 484
- US-A- 2 758 710
- US-A- 3 162 306
- US-A- 3 485 349
- US-A- 3 743 084

## Beschreibung

Die Erfindung betrifft eine Vorratseinrichtung für Tupfer, insbesondere sterilisierte medizinische Tupfer.

Medizinische Tupfer sind in Sammelpackung sterilisiert bevorratet. Sie werden einzeln verwendet und einzeln aus einer Sammelpackung entnommen. Nach Öffnung der Sammelpackung und Entnahme der ersten Tupfer ist die Sterilität der übrigen bevorrateten Tupfer nicht mehr sichergestellt, so daß diese resterilisiert werden müssen. Auch die Anordnung der Sammelpackung in einer weiteren Umverpackung kann die Sterilität der in der einmal geöffneten Sammelpackung befindlichen Tupfer nicht sicherstellen.

In WO-A-95 18046 wird eine Vorratseinrichtung für medizinische Tupfer beschrieben, bei der die Umhüllung aus zwei aneinander angeordneten und bakteriendicht durch eine Kleberschicht lösbar miteinander verschlossenen Kammern besteht. Dabei ist die Deckschicht mit einer Griffhilfe zum Abziehen derselben von der Basisschicht versehen, um den Kammerinhalt zugänglich zu machen. Eine Vielzahl von Kammern ist benachbart angeordnet, und die Umhüllung ist gerollt oder in Zickzack-Lage bevorratbar.

Bei einer in der US-A-3 743 084 beschriebenen Verpackung handelt es sich um eine weitgehend biegesteife Verbindung zweier Folien, die weder faltbar noch durch die langgestreckt ausgebildeten Kammern rollbar ist.

Der Erfindung liegt die Aufgabe zugrunde, eine Vorratseinrichtung zu schaffen, die die Entnahme einzelner sterilisierter Tupfer bei Sicherstellung der Sterilität der übrigen in der Vorratseinrichung bevorrateten Tupfer gestattet. Außerdem soll der Platzbedarf der Vorratseinrichtung minimiert sein.

Diese Aufgabe wird bei einer Vorratseinrichtung nach dem Oberbegriff des Anspruchs 1 durch dessen kennzeichnende Merkmale gelöst.

Die erfindungsgemäße Vorratseinrichtung weist eine Umhüllung aus einzelnen bakteriendicht verschlossenen Kammern für die Tupfer auf, die benachbart angeordnet sind. Die Größe der Kammern ist durch die Anzahl und die Größe der jeweils in diesen bevorrateten Tupfer bestimmt, wobei zur Sicherstellung einer absoluten Sterilität lediglich ein Tupfer in einer Kammer bevorratet sein sollte, so daß jeder Tupfer sterilisiert aus der Vorratseinrichtung entnommen werden kann. Für medizinische Bereiche, in denen eine große Anzahl von Tupfern verbraucht wird, ist die Sterilität eines zweiten, in der gleichen Kammer bevorrateten und nach der Entnahme des ersten Tupfers kurzzeitig verbleibenden Tupfers ebenfalls gegeben, so daß in einer Vorratseinrichtung für in derartigen Bereichen verwendete Tupfer durchaus auch zwei Tupfer in einer Kammer bevorratet sein können. Die Umhüllung muß so flexibel sein, daß sie gerollt oder in Zickzack-Lage bevorratbar ist.

Dazu kann die Umhüllung aus einer flexiblen Basisschicht und einer mit dieser verbundenen flexiblen Deckschicht gebildet sein, von denen eine Schicht gegenüber der anderen Schicht zur Bildung der Kammern bereichsweise aufgewölbt ist.

Zwischen den Kammern können Sollzerreißstege ausgebildet sein, um die Entnahme eines Tupfers aus der Vorratseinrichtung zu erleichtern und um die Handhabbarkeit derselben insgesamt zu verbessern.

Die Kammern können in einer Reihe oder in mehreren parallelen Reihen ausgebildet sein, wobei zur Platzeinsparung die Kammern benachbarter Reihen versetzt zueinander angeordnet sein können, um die Zwischenräume zu minimieren.

Die Kammern können auf einfache Weise durch Aufnahmemulden in der Basisschicht gebildet sein, die durch die Deckschicht abgedeckt werden. Dabei ist es auch möglich, daß jede Aufnahmemulde durch eine vereinzelte Deckelschicht verschlossen ist.

Die Aufnahmemulden können dabei durch einen Tiefziehprozeß hergestellt sein, und die Anordnung der Deckschicht oder der einzelnen Deckelschichten kann durch Schweißen oder Siegeln erfolgen, um eine stoffschlüssige Verbindung zwischen den Schichten herzustellen.

Die Handhabbarkeit der erfindungsgemäßen Vorratseinrichtung kann wesentlich verbessert werden, wenn diese gerollt oder in Zickzack-Lage in einem Spender in Form einer Umverpackung angeordnet ist, der eine Entnahmeöffnung für eine oder gegebenenfalls mehrere nebeneinander angeordnete Kammern aufweist. Der Spender kann aufrecht oder liegend zum Einsatz kommen.

Die Erfindung wird nachstehend anhand eines Ausführungsbeispiels erläutert. Die zugehörige Zeichnung zeigt:
- Figur 1:: einen Schnitt durch einen Abschnitt einer erfindungsgemäßen Vorratseinrichtung und
- Figur 2:: eine perspektivische Ansicht der Vorratseinrichtung.

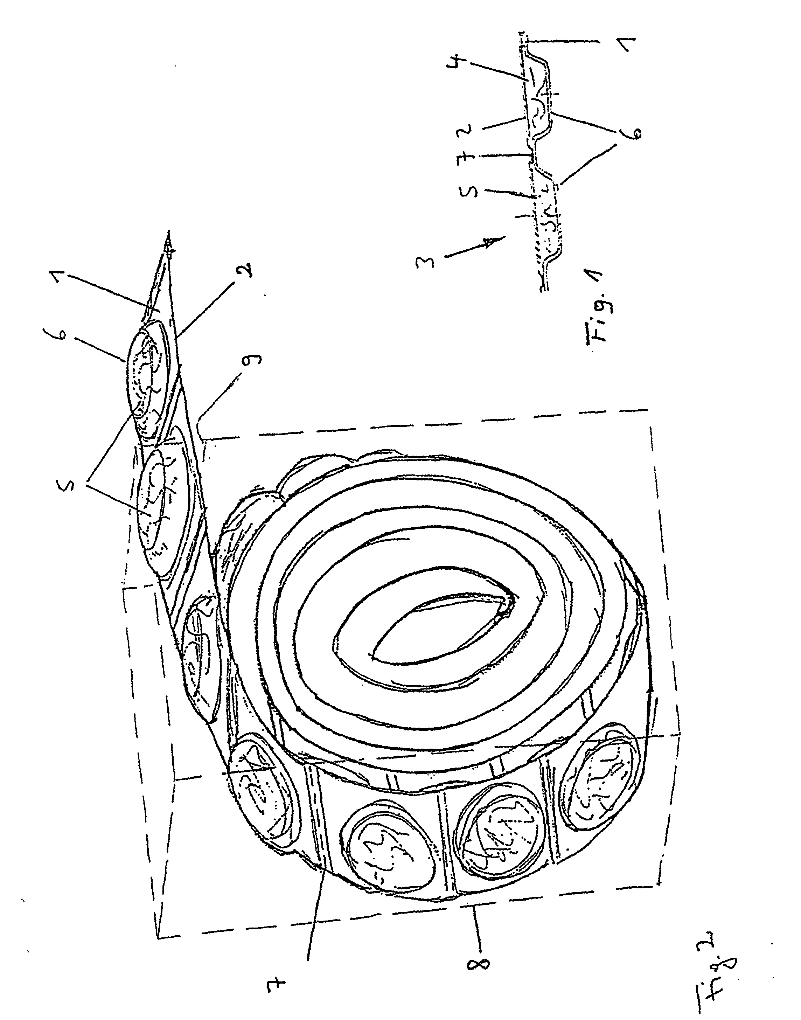

Die in Figur 1 im Schnitt dargestellte Vorratseinrichtung weist eine aus einer flexiblen Basisschicht 1 aus Polyamid/Polyethylen und einer flexiblen Deckschicht 2 ( aus Sterilisationskraftpapier ) bestehende Umhüllung 3 auf, in der Kammern 4 für jeweils einen Tupfer 5 ausgebildet sind. Die Kammern 4 sind durch in der Basisschicht 1 ausgebildete Aufnahmemulden 6 und die über diesen angeordnete Deckschicht 2 gebildet und durch diese Basisschicht 1 in den Bereichen zwischen den Aufnahmemulden 6 verschweißt. Damit sind die in den Kammern 4 bevorrateten Tupfer 5 bakteriendicht verschlossen. Die Schweißstellen sind zugleich als Sollzerreißstellen 7 mit verminderter Deckschichtdicke ausgebildet. Die Kammern 4 sind hintereinander in einer Reihe angeordnet.

Figur 2 zeigt die Vorratseinrichtung mit gerollter Umhüllung 3. Gestrichelt ist ein Spender 8 angedeutet, der die gerollte Umhüllung 3 umgibt und eine Entnahmeöffnung 9 aufweist, durch die die Umhüllung 3 gezogen werden und kammerweise an den Sollzerreißstegen 7 vereinzelt werden kann. Die Öffnung der Kammern 4 erfolgt durch Fingerdruck auf die Deckschicht, die dadurch einreißt.

## Patentansprüche

1. Vorratseinrichtung für medizinische Tupfer mit einer Umhüllung (3) für diese, wobei die Umhüllung eine flexible Basisfolie (1) und eine flexible Deckfolie (2) aufweist, zwischen denen benachbart angeordnete Kammern (4) ausgebildet sind, deren Größe durch die Anzahl und die Größe der jeweils in diesen bevorrateten Tupfer (5) bestimmt ist, und die Umhüllung gerollt oder in Zickzack-Lage bevorratbar ist, **dadurch gekennzeichnet, daß** die beiden Folien (1, 2) in den Bereichen zwischen den Kammern (4) stoffschlüssig durch Siegeln oder Schweißen miteinander verbunden und die Kammern (4) durch Fingerdruck auf die Deckfolie (2) aufbrechbar sind.

2. Vorratseinrichtung nach Anspruch 1, **dadurch gekennzeichnet, daß** die Kammern (4) durch Aufnahmemulden (6) bildende beabstandete, aufgewölbte Bereiche in der Basisfolie (1) gebildet sind, die durch die Deckfolie (2) verschlossen sind.

3. Vorratseinrichtung nach Anspruch 1 oder 2, **dadurch gekennzeichnet, daß** zwischen den Kammern (4) Sollzerreißstege (7) ausgebildet sind.

4. Vorratseinrichtung nach einem der vorhergehenden Ansprüche, **dadurch gekennzeichnet, daß** die Kammern (4) in einer Reihe oder in mehreren parallelen Reihen angeordnet sind.

5. Vorratseinrichtung nach einem der vorhergehenden Ansprüche, **dadurch gekennzeichnet, daß** die Umhüllung (3) gerollt oder in Zickzacklage in einem Spender (8) mit einer Entnahmeöffnung (9) in Form einer Umverpackung angeordnet ist.

## Claims

1. Storage device for medical swabs having an envelope (3) for said swabs, said envelope having a flexible base film (1) and a flexible covering film (2) between which are formed adjacent chambers (4), the size of said chambers (4) being determined by the number and size of the respective swabs (5) stored in said chambers, said envelope being storable in rolled or concertina fold position, **characterized in that** in the areas between the chambers (4) the said two films (1, 2) are connected with each other by sealing or welding so as to be bonded, and that the chambers (4) can be broken open by exerting finger pressure on the covering film (2).

2. Storage device according to Claim 1, **characterized in that** the chambers (4) are formed by spaced-apart, convex areas in the base film (1) which form receiving troughs (6) and which are closed by the cover film (2).

3. Storage device according to Claim 1 or 2, **characterized in that** strips (7) intended to be torn through are provided between the chambers (4).

4. Storage device according to any of the preceding claims, **characterized in that** the chambers (4) are disposed in a row or in several parallel rows.

5. Storage device according to any of the preceding claims, **characterized in that** the envelope (3) is disposed in rolled or concertina fold position in a dispenser (8) having a withdrawal opening (9), said dispenser (8) being in the form of a secondary pack.

## Revendications

1. Système de réservoir pour tampons à usage médical avec un emballage (3) pour ceux-ci, l'emballage comprenant une feuille de base (1) et une feuille de recouvrement (2) souples entre lesquelles sont réalisées des chambres (4) contiguës dont la taille est définie par le nombre et la taille des tampons (5) conservés à chaque fois dans celles-ci et l'emballage pouvant être conservé en rouleau ou en zigzag, **caractérisé en ce que** les deux feuilles (1, 2), par concordance des matières, sont reliées entre elles par scellage ou soudage dans les zones situées entre les chambres (4) et **en ce que** les chambres (4) peuvent s'ouvrir par pression du doigt sur la feuille de recouvrement (2).

2. Système de réservoir selon la revendication 1, **caractérisé en ce que** les chambres (4) sont réalisées par des zones bombées, distancées formant des logements en creux (6) dans la feuille de base (1), qui sont fermées par la feuille de recouvrement (2).

3. Système de réservoir selon la revendication 1 ou 2, **caractérisé en ce que** des ponts destinés à la rupture (7) sont réalisés entre les chambres (4).

4. Système de réservoir selon l'une des revendications précédentes, **caractérisé en ce que** les chambres (4) sont disposées en une rangée ou en plusieurs rangées parallèles.

5. Système de réservoir selon l'une des revendications précédentes, **caractérisé en ce que** l'emballage (3), en rouleau ou en zigzag, est placé dans un distributeur (8) muni d'un orifice de sortie (9), réalisé sous forme de suremballage.
